# FASCICULE DE BREVET EUROPEEN

(11) **EP 0 695 139 B1**
(45) Date de publication et mention de la délivrance du brevet: **01.12.1999**
(21) Numéro de dépôt: 94913678.2
(22) Date de dépôt: 21.04.1994
(51) Int. Cl.: A61B 5/055, A61B 5/0404, A61B 5/024, A61B 5/0428, G01R 33/48

(54) **DISPOSITIF CAPTEUR POUR ELECTROCARDIOGRAMME**
EKG-SENSOR
SENSOR DEVICE FOR ELECTROCARDIOGRAM

(30) Priorité: 22.04.1993 FR 9304918
(43) Date de publication de la demande: 07.02.1996
(73) Titulaire: O.D.A.M. - OFFICE DE DISTRIBUTION D'APPAREILS MEDICAUX, 67160 Wissembourg (FR)
(72) Inventeur: FELBLINGER, Jacques, CH-3125 Toffen (CH); BOESCH, Chris, CH-3600 Thun (CH); MULLER, Gérard, F-57410 Rohrbach-les-Bitche (FR); KRAEMER, Michel, F-67360 Durrenbach (FR)
(74) Mandataire: Nuss, Pierre
(86) Numéro de dépôt international: FR9400454
(87) Numéro de publication internationale: WO9423648

(56) Documents cités:
- EP-A- 0 132 785
- EP-A- 0 170 448
- EP-A- 0 173 130
- EP-A- 0 178 990
- EP-A- 0 498 996
- WO-A-88/05282
- US-A- 4 181 134

## Description

La présente invention concerne le domaine du prélèvement et de la mesure de signaux biologiques et de la surveillance de patients, notamment de patients sous examen RMN (Résonance Magnétique Nucléaire) par exemple dans un Imageur à Résonance Magnétique (IRM), et a pour objet un dispositif capteur pour électrocardiogramme.

Actuellement, les signaux d'électrocardiogramme des patients soumis à un examen RMN sont prélevés au moyen d'électrodes à conducteurs métalliques et transmis à un appareil de visualisation et/ou de surveillance disposé dans l'enceinte blindée ou à l'extérieur au moyen de câbles électriques de grande longueur, le cas échéant blindés et torsadés.

Toutefois, ces câbles électriques, en formant antennes, perturbent l'environnement électromagnétique de l'appareil à RMN et faussent les mesures et, dans le cas d'un imageur, les reconstitutions virtuelles (images) obtenues par ce dernier.

A l'inverse, les gradients de champ, les champs radiofréquence et les phénomènes liés aux commutations entre bobines émettrices et réceptrices au cours d'une expérience au type RMN perturbent fortement la transmission des signaux de faible puissance relevés au niveau du coeur et peuvent, par la génération d'artefacts importants, rendre ces derniers totalement inexploitables, le patient étant disposé à l'intérieur même de l'aimant principal de l'appareil à RMN.

De plus, les mouvements éventuels du patient (notamment respiration) entraînent des mouvements desdits câbles de transmission électrique dans le champ résident, d'où résulte automatiquement une induction de potentiels générateurs d'artefacts.

En outre, les phénomènes nuisibles précités sont fortement amplifiés lorsque les câbles de transmission présentent une ou plusieurs boucles.

En effet, l'énergie emmagasinée par le ou les câble(s), soumis à des champs électromagnétiques de forte intensité, au niveau desdites boucles ou du câble peut être assez importante pour provoquer un échauffement important dudit câble, pouvant provoquer des brûlures au niveau de la peau du patient, sur lequel repose une partie des câbles, notamment lorsqu'on traverse une antenne émettrice.

Par ailleurs, le placement des électrodes sur le patient dans la région du coeur nécessite, en vue d'une mise en place correcte, l'intervention d'une personne spécialisée, chacune desdites électrodes devant être positionnée précisément de manière individuelle.

Il a été proposé, en vue de tenter de pallier certains de ces inconvénients, de mettre en oeuvre des algorithmes de correction des artefacts générés par les gradients et les champs radiofréquence.

Toutefois, ces algorithmes ne sont adaptés qu'à un type défini d'appareils à RMN, à une configuration donnée de bobines et souvent à une séquence RMN particulière, d'où résulte un manque de souplesse important lors de leur application.

En outre, ils ne permettent pas de résoudre les problèmes de brûlures, ni les problèmes d'induction de potentiels du fait des mouvements des câbles ou encore les problèmes de positionnement des électrodes.

Par le document EP-A-0 173 130, on connaît déjà un capteur d'électrocardiogramme destiné à être mis en oeuvre à l'intérieur d'un appareil à RMN.

Ce capteur connu comporte notamment plusieurs électrodes destinées à être disposées dans la région du coeur et reliées par des câbles de transmission électriques à un dispositif convertisseur électro-optique dont la sortie est connectée, au moyen d'une ligne de transmission optique, à un dispositif de surveillance et d'affichage situé à l'extérieur de la cage de Faraday entourant l'imageur RMN.

Néanmoins, dans ce dispositif la transmission des signaux entre les électrodes et le convertisseur s'effectue au moyen de câbles électriques générant les inconvénients décrits ci-dessus, notamment la perturbation des signaux transmis sous forme électrique et les risques de brûlures auxquels les patients sont exposés.

Enfin, par le document EP-A-0 170 448 a pour objet un moniteur d'électrocardiogramme portable, de faible encombrement et destiné à des interventions d'urgence, qui est essentiellement constitué par trois électrodes sèches, par une unité de traitement et par un module d'affichage.

Toutefois, ce dispositif ne présente aucune des caractéristiques requises pour une mise en oeuvre dans un environnement électromagnétique chargé et sensible, tel que l'intérieur d'un aimant d'un imageur RMN, ni pour la transmission des signaux recueillis à l'extérieur de la cage de Faraday entourant ledit imageur.

La présente invention a notamment pour but de pallier les inconvénients évoqués précédemment.

A cet effet, elle a pour objet un dispositif capteur pour électrocardiogramme présentant les caractéristiques mentionnées dans la revendication 1.

L'invention sera mieux comprise grâce à la description ci-après, qui se rapporte à un mode de réalisation préféré, donné à titre d'exemple non limitatif, et expliqué avec référence aux dessins schématiques annexés, dans lesquels :
la figure 1 est une vue schématique, en élévation latérale, du dispositif capteur selon l'invention :
la figure 2 est un schéma fonctionnel du dispositif capteur représenté à la figure 1 :
la figure 3 est une représentation schématique montrant la mise en oeuvre du dispositif capteur représenté à la figure 1 :
la figure 4 représente un signal d'électrocardiogramme obtenu au moyen d'un dispositif capteur conforme à l'invention, et
la figure 5 représente un signal d'électrocardiogramme obtenu au moyen d'un capteur usuel, dans des conditions identiques à celles ayant conduit à l'électrocardiogramme de la figure 4.

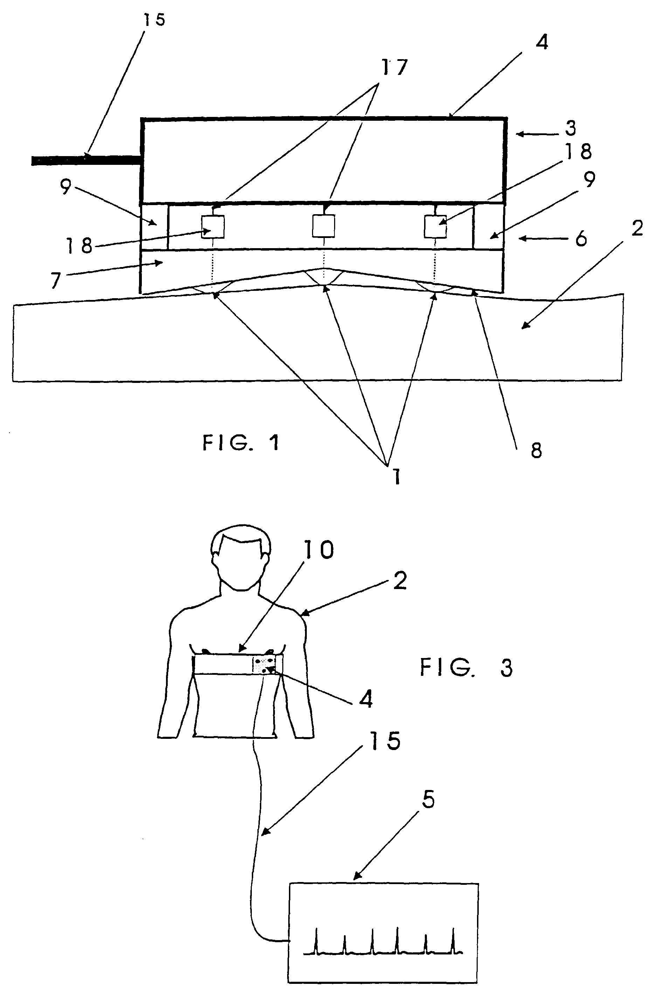

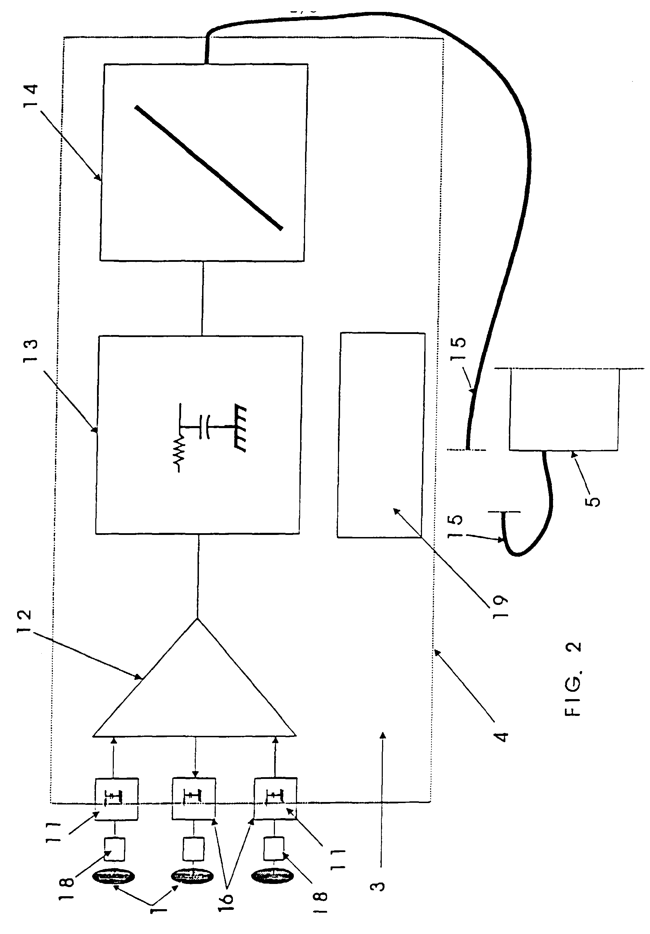

Conformément à l' invention et comme le montrent les figures 1 et 2 des dessins annexés, le dispositif capteur est principalement constitué d'une part, par au moins deux électrodes 1 conductrices non métalliques, destinées à être appliquées sur la peau du patient 2, d'autre part, par un module 3 de filtrage, d'amplification et de conversion électrooptique des signaux électriques du coeur relevés au moyen des électrodes réceptrices 1, disposé dans un boîtier blindé 4 formant cage de Faraday et relié, par voie optique, à un appareil 5 de visualisation et/ou de contrôle et, enfin, par un corps support 6 en un matériau amagnétique non métallique. portant les électrodes 1 et le boîtier blindé 4 contenant le module 3 et maintenant rigidement ledit boîtier blindé 4 à une distance constante de la peau du patient 2.

Les signaux électrocardiographiques relevés par les électrodes 1 sont, par conséquent, immédiatement amplifiés et filtrés à l'aide d'un module 3 adéquat, totalement isolé de l'environnement électromagnétique extérieur, et, de même, transformé et transmis sous forme optique pratiquement à partir de leur endroit de prélèvement.

Selon une première caractéristique de l'invention, représentée à la figure 1 des dessins annexés, le corps support 6 est composé d'une embase 7 sur laquelle sont montées de manière fixe les électrodes réceptrices 1, en étant proéminentes du côté 8 appliqué contre la peau du patient 2, et au moins un élément 9 séparateur ou d'espacement maintenant rigidement le boîtier blindé 4 contenant le module 3 à une distance constante de l'embase 7 et donc de la peau du patient 2.

L'unique élément métallique du dispositif capteur, à savoir le boîtier blindé 4, ne sera, par conséquent, jamais en contact direct avec le patient 2, ce qui évite tout risque de brûlure.

En outre, les électrodes l étant fixées sur l'embase 7, les emplacements relatifs des unes par rapport aux autres sont figés en étant écartés d'une distance fonction de la taille du patient et il suffit de positionner ladite embase 7 à proximité du coeur pour que les électrodes 1 soient placées de manière satisfaisante.

Le corps support 6 peut être avantageusement réalisé en un matériau tel que le téflon ou le polyméthacrylate de méthyle (également connu sous la dénomination "plexiglass"), en présentant une structure aux formes arrondies, sans arêtes.

De même, les électrodes 1, avantageusement au nombre de trois, sont préférentiellement réalisées en un matériau conducteur choisi dans le groupe formé par le carbone, les composés de carbone et les matières plastiques chargées et insensible aux champs électromagnétiques.

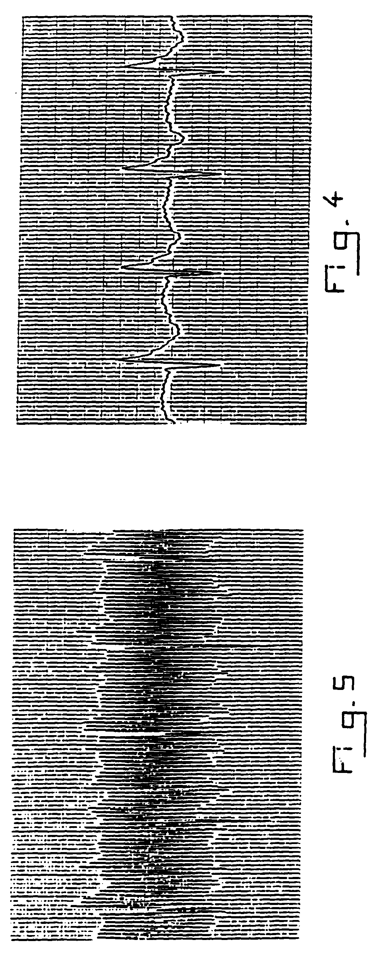

Conformément à un mode de réalisation préféré de l'invention, représenté à la figure 3 des dessins annexés et afin d'assurer un positionnement ferme des électrodes 1 et de garantir un prélèvement des signaux électriques cardiaques aussi près que possible du coeur, il est prévu une ceinture 10 ou un harnais en un matériau amagnétique, éventuellement élastique, pourvu d'un moyen de fermeture rapide et de réglage de la longueur et traversant le corps support 6 ou au moins une anse solidaire dudit corps support 6.

Ainsi, l'embase 7 du corps support 6 et donc les électrodes 1 seront, en permanence, appliqués à force contre la peau du patient au niveau de la région du coeur.

Toutefois, tout autre moyen de fixation adéquat peut également être envisagé, à condition qu'il consiste en un matériau amagnétique.

Comme le montre la figure 2 des dessins annexés, le module 3 de filtrage, d'amplification et de conversion peut être composé, pour l'essentiel, d'une part, d'unités 11 de filtrage haute-fréquence associées chacune à une des électrodes 1, d'autre part, d'un circuit 12 d'amplification différentielle associé à un filtre 13 passe-bas et, enfin, d'un transducteur 14 électrooptique relié, par un conducteur optique 15, à un appareil 5 de visualisation et/ou de contrôle, la transmission optique des signaux étant opérée, par exemple, par modulation de fréquence ou de largeur d'impulsions.

Chacune des unités 11 de filtrage haute-fréquence assurant l'interface entre les électrodes 1 et le module 3, pourra avantageusement être disposée dans un boîtier blindé 16 formant cage de Faraday et les liaisons électriques entre lesdites électrodes 1 et les unités 11 de filtrage du module 3 seront préférentiellement réalisées au moyen de fils rigides 17 de faible longueur, le cas échéant blindés et intégrant chacun une résistance de limitation 18, limitant les interférences des ondes électromagnétiques extérieures et évitant toute formation de boucles et risque de brûlure.

Le circuit 12 d'amplification différentielle pourra présenter, par exemple, un gain d'environ 300 à 500 et l'une des trois électrodes l faisant partie du dispositif capteur selon l'invention, pourra être utilisée pour la réinjection du mode commun (les deux autres fonctionnent en réception), permettant de s'affranchir des signaux parasites, notamment basse fréquence, relevés par les deux électrodes réceptrices 1.

Le filtre 13 pourra avantageusement présenter une fréquence de coupure de l'ordre de 20 Hz.

L'appareil 5 de visualisation et/ou de contrôle pourra être disposé soit dans l'enceinte de l'appareil à RMN (cage de Faraday), soit à l'extérieur de celle-ci et comportera une unité de reconversion opto-électrique, un écran de visualisation et/ou un enregistreur et/ou un module de détection ou d'analyse des complexes QRS ou d'un autre paramètre du signal d'électrocardiogramme, permettant de déclencher ou de commander un ou plusieurs appareils d'analyse, de visualisation et/ou de test du patient.

L'appareil 5 pourra, par exemple, consister en un moniteur de surveillance des paramètres physiologiques vitaux d'un patient en cours d'examen dit IRM, du type de celui faisant l'objet du dépôt français n° 9014846 du 23 novembre 1990 au nom de la demanderesse.

Selon une première variante de réalisation de l'invention, le module 3 comprend, en outre, en vue de son alimentation électrique, une batterie 18 ou un accumulateur rechargeable longue durée et de type amagnétique, un conducteur optique, associé à un interrupteur à commande optique disposé dans le boîtier, pouvant permettre de contrôler le fonctionnement et l'alimentation dudit module 3 de filtrage, d'amplification et de conversion et, le cas échéant, le réglage des différents composants (11 à 14) de ce dernier (non représenté).

Conformément à une seconde variante de réalisation de l'invention, l'alimentation en énergie du module 3 de filtrage, d'amplification et de conversion est réalisée au moyen d'un conducteur optique coopérant avec une cellule photovoltaïque ou un dispositif similaire disposé dans le boîtier blindé 4.

L'amélioration de la qualité des signaux d'électrocardiogramme relevés, résultant de la mise en oeuvre de l'invention, devient parfaitement apparente en comparant les courbes de signaux des figures 4 et 5 entre elles, ces signaux ayant été relevés sur un patient soumis à un examen RMN au moyen d'une antenne corps entier.

## Revendications

1. Dispositif capteur pour électrocardiogramme, destiné à être mis en oeuvre dans un environnement électromagnétique chargé et sensible, sur un patient soumis à un examen RMN à l'intérieur d'un appareil à résonance magnétique nucléaire, et plus particulièrement sur un patient à l'intérieur du tunnel de l'aimant d'un IRM, caractérisé en ce qu'il est principalement constitué, d'une part, par au moins deux électrodes (1) conductrices non métalliques, destinées à être appliquées sur la peau du patient (2), d'autre part, par un module (3) de filtrage, d'amplification et de conversion électrooptique des signaux électriques du coeur relevés au moyen des électrodes réceptrices (1), disposé dans un boîtier blindé (4) formant cage de Faraday et relié, par voie optique, à un appareil (5) de visualisation et/ou de contrôle et, enfin, par un corps support (6) en un matériau amagnétique non métallique, portant les électrodes (1) et le boîtier blindé (4) contenant le module (3) et maintenant rigidement ledit boîtier blindé (4) à une distance constante de la peau du patient (2).

2. Dispositif capteur selon la revendication 1, caractérisé en ce que le corps support (6) est composé d'une embase (7) sur laquelle sont montées de manière fixe les électrodes réceptrices (1), en étant proéminentes du côté (8) appliqué contre la peau du patient (2), et au moins un élément (9) séparateur ou d'espacement maintenant rigidement le boîtier blindé (4) contenant le module (3) à une distance constante de l'embase (7).

3. Dispositif capteur selon l'une quelconque des revendications 1 et 2, caractérisé en ce que les électrodes (1), avantageusement au nombre de trois, sont réalisées en un matériau conducteur choisi dans le groupe formé par le carbone, les composés de carbone et les matières plastiques chargées.

4. Dispositif capteur selon l'une quelconque des revendications 1 à 3, caractérisé en ce qu'il comprend une ceinture (10) ou un harnais en un matériau amagnétique, éventuellement élastique, pourvu d'un moyen de fermeture rapide et de réglage de la longueur et traversant le corps support (6) ou au moins une anse solidaire dudit corps support (6).

5. Dispositif capteur selon l'une quelconque des revendications 1 à 4, caractérisé en ce que le module (3) de filtrage, d'amplification et de conversion est composé, d'une part, d'unités (11) de filtrage haute-fréquence associées chacune à une des électrodes (1), d'autre part, d'un circuit (12) d'amplification différentielle associé à un filtre (13) passe-bas et, enfin, d'un transducteur (14) électro-optique relié, par un conducteur optique (15), à un appareil (5) de visualisation et/ou de contrôle, la transmission optique des signaux étant opérée, par exemple, par modulation de fréquence ou de largeur d'impulsions.

6. Dispositif capteur selon l'une quelconque des revendications 1 à 5, caractérisé en ce que les liaisons électriques entre les électrodes (1) et les unités (11) de filtrage du module (3) sont réalisées au moyen de fils rigides (17) de faible longueur, le cas échéant blindés et intégrant chacun une résistance de limitation (18).

7. Dispositif capteur selon l'une quelconque des revendications 5 et 6, caractérisé en ce qu'il comporte trois électrodes (1), l'une d'entre elles étant utilisée pour la réinjection du mode commun.

8. Dispositif capteur selon l'une quelconque des revendications 1 à 7, caractérisé en ce que le module (3) comprend, en outre, une batterie (18) ou un accumulateur rechargeable longue durée et de type amagnétique, un conducteur optique, associé à un interrupteur à commande optique disposé dans le boîtier, pouvant permettre de contrôler le fonctionnement et l'alimentation dudit module (3) de filtrage, d'amplification et de conversion et, le cas échéant, le réglage des différents composants (11 à 14) de ce dernier.

9. Dispositif capteur selon l'une quelconque des revendications 1 à 7, caractérisé en ce que l'alimentation en énergie du module (3) de filtrage, d'amplification et de conversion est réalisée au moyen d'un conducteur optique coopérant avec une cellule photovoltaïque ou un dispositif similaire disposé dans le boîtier blindé.

## Claims

1. Sensor device for an electrocardiogram intended to be used in a charged and sensitive electromagnetic environment on a patient subjected to NMR examination within a nuclear magnetic resonance apparatus and more particularly on a patient within the tunnel of the magnet of an MRI, characterised in that it consists mainly, on the one hand, of at least two non-metallic conducting electrodes (1) intended to be applied to the patient's skin (2), on the other hand, of a module (3) for the filtering, amplification and electrooptical conversion of the electrical signals from the heart taken by means of receiving electrodes (1) disposed in a shielded casing (4) forming a Faraday cage and optically connected to a display and/or control apparatus (5) and, finally, of a supporting member (6) made of a non-metallic non-magnetic material, carrying the electrodes (1) and the shielded casing (4) containing the module (3) and rigidly holding said shielded casing (4) at a constant distance from the patient's skin (2).

2. Sensor device according to claim 1, characterised in that the supporting member (6) is composed of a base (7), on which are rigidly mounted the receiving electrodes (1) which project on the side (8) applied to the patient's skin (2), and at least one separating or spacing element (9) rigidly holding the shielded casing (4) containing the module (3) at a constant distance from the base (7).

3. Sensor device according to any one of claims 1 and 2, characterised in that the electrodes (1), of which there are advantageously three, are produced from a conductive material selected from the group formed by carbon, carbon compounds and reinforced plastics materials.

4. Sensor device according to any one of claims 1 to 3, characterised in that it comprises a belt (10) or a harness made of an optionally elastic non-magnetic material provided with a means for rapid closure and control of the length and traversing the supporting member (6) or at least one handle integral with said supporting member (6).

5. Sensor device according to any one of claims 1 to 4, characterised in that the module (3) for filtering, amplification and conversion is composed, on the one hand, of high-frequency filtering units (11) each associated with one of the electrodes (1), on the other hand, of a differential amplification circuit (12) associated with a low-pass filter (13) and, finally, of an electrooptical transducer (14) connected, via an optical conductor (15), to a display and/or control apparatus (5), the optical transmission of the signals being effected, for example, by frequency or pulse width modulation.

6. Sensor device according to any one of claims 1 to 5, characterised in that the electrical connections between the electrodes (1) and the filtering units (11) of the module (3) are produced by means of rigid wires (17) of short length, optionally shielded and each comprising a current-limiting resistor (18).

7. Sensor device according to any one of claims 5 and 6, characterised in that it comprises three electrodes (1), one of them being used for reinjection of the common mode.

8. Sensor device according to any one of claims 1 to 7, characterised in that the module (3) also comprises a battery (18) or a long-life rechargeable accumulator of the non-magnetic type, an optical conductor associated with an optically controlled switch disposed in the casing allowing control of the operation and supply of said filtering, amplification and conversion module (3) and, if applicable, controlling the various components (11 to 14) thereof.

9. Sensor device according to any one of claims 1 to 7, characterised in that energy is supplied to the filtering, amplification and conversion module (3) by means of an optical conductor cooperating with a photovoltaic cell or a similar device disposed in the shielded casing.

## Patentansprüche

1. Sensorvorrichtung für Elektrokardiogramm, bestimmt für den Einsatz in einer geladenen und empfindlichen elektromagnetischen Umgebung, an einem Patienten, der einer NMR-Untersuchung unterzogen wird im Inneren eines Gerätes mit magnetischer Kernresonanz, und insbesondere an einem Patienten im Inneren des Tunnels des Magneten eines MRI, dadurch gekennzeichnet, daß sie im wesentlichen gebildet ist durch einerseits wenigstens zwei nicht-metallische leitfähigen Elektroden (1), die zur Anwendung auf der Haut des Patienten (2) bestimmt sind, andererseits durch ein Modul (3) zur Filterung, Verstärkung und elektrooptischen Umwandlung der elektrischen Signale des Herzens, aufgenommen durch die Empfängerelektroden (1), die in einem abgeschirmten, einen Faraday-Käfig bildenden Gehäuse (4) angeordnet und auf optischem Weg mit einem Gerät (5) zur Visualisierung und/oder Kontrolle verbunden sind, und schließlich durch einen Trägerkörper (6) aus einem nicht-metallischen, unmagnetischen Material, der die Elektroden (1) und das abgeschirmte, das Modul (3) enthaltende Gehäuse (4) trägt und das abgeschirmte Gehäuse (4) starr in einer konstanten Entfernung von der Haut des Patienten (2) hält.

2. Sensorvorrichtung nach Anspruch 1, dadurch gekennzeichnet, daß der Trägerkörper (6) aus einem Sockel (7), auf dem die Empfängerelektroden (1) fest montiert sind, indem sie von der Seite (8), die der Haut des Patienten (2) zugewendet ist, vorspringend sind, und wenigstens einem Trenn- oder Abstandselement (9) gebildet ist, welches das abgeschirmte Gehäuse (4), das das Modul (3) enthält, starr in einer konstanten Entfernung zum Sockel (7) hält.

3. Sensorvorrichtung nach Anspruch 1 oder 2, dadurch gekennzeichnet, daß die Elektroden (1), vorteilhafterweise drei an der Zahl, aus einem leitendem Material bestehen, ausgewählt aus der Gruppe gebildet durch Kohlenstoff, die Kohlenstoffverbindungen und die mit Füllstoffen versehenen Kunststoffe.

4. Sensorvorrichtung nach einem der Ansprüche 1 bis 3, dadurch gekennzeichnet, daß sie einen Gürtel (10) oder eine Manschette aus einem unmagnetischen, gegebenenfalls elastischen Material aufweist, versehen mit einem Mittel zum schnellen Verschließen und zur Einstellung der Länge, das den Trägerkörper (6) oder wenigstens einen Griff, der mit dem Trägerkörper (6) verbunden ist, durchquert.

5. Sensorvorrichtung nach einem der Ansprüche 1 bis 4, dadurch gekennzeichnet, daß das Modul (3) zur Filterung, Verstärkung und Umwandlung gebildet ist einerseits aus Einheiten (11) zur Hochfrequenz-Filterung, die jeweils mit einer der Elektroden (1) verbunden sind, und anderseits einem Stromkreis (12) zur Differentialverstärkung, der mit einem Tiefpaß-Filter (13) verbunden ist, und schließlich einem elektrooptischen Wandler (14), der durch einen optischen Leiter (15) mit einem Gerät (5) zur Visualisierung und/oder Kontrolle verbunden ist, wobei die optische Übertragung der Signale, beispielsweise, durch Modulation der Impulsfrequenz oder -breite durchgeführt wird.

6. Sensorvorrichtung nach einem der Ansprüche 1 bis 5, dadurch gekennzeichnet, daß die elektrischen Verbindungen zwischen den Elektroden (1) und den Einheiten (11) zur Filterung des Moduls (3) mittels steifer Drähte (17) von geringer Länge, die gegebenenfalls abgeschirmt sind und jeweils einen Begrenzungswiderstand beinhalten, hergestellt sind.

7. Sensorvorrichtung nach Anspruch 5 oder 6, dadurch gekennzeichnet, daß sie drei Elektroden (1) aufweist, wobei eine von ihnen für die Rückkopplung des Gleichtakts verwendet wird.

8. Sensorvorrichtung nach einem der Ansprüche 1 bis 7, dadurch gekennzeichnet, daß das Modul (3) außerdem eine Batterie (18) oder einen wiederaufladbaren, langlebigen Akkumulator der unmagnetischen Art, einen optischen Leiter, der mit einem in dem Gehäuse angeordneten Unterbrecher mit optischer Steuerung verbunden ist, der die Kontrolle des Betriebs und der Speisung des Moduls (3) zur Filterung, Verstärkung und Umwandlung und gegebenenfalls die Steuerung der verschiedenen Bestandteile (11 bis 14) von letzterem ermöglichen kann, umfaßt.

9. Sensorvorrichtung nach einem der Ansprüche 1 bis 7, dadurch gekennzeichnet, daß die Speisung des Moduls (3) zur Filterung, Verstärkung und Umwandlung mit Energie mittels eines optischen Leiters bewirkt ist, der mit einer in dem abgeschirmten Gehäuse angeordneten photovoltaischen Zelle oder einer ähnlichen Vorrichtung zusammenwirkt.
